# EUROPÄISCHE PATENTANMELDUNG

(11) **EP 4 247 008 A1**
(43) Veröffentlichungstag der Anmeldung: **20.09.2023**
(21) Anmeldenummer: 23160742.5
(22) Anmeldetag: 08.03.2023
(51) Int. Cl.: H04R 25/00, A61B 5/103, A61B 5/00

(54) **VERFAHREN ZUM BETRIEB EINES HÖRGERÄTS**

(30) Priorität: 15.03.2022 DE 102022202568
(71) Anmelder: Sivantos Pte. Ltd., Singapore 539775 (SG)
(72) Erfinder: KRIEG, Julius, 91058 Erlangen (DE); KUKLA, Christoph, 91058 Erlangen (DE)
(74) Vertreter: FDST Patentanwälte

(57) **Zusammenfassung**

Die Erfindung betrifft ein Verfahren (36) zum Betrieb eines Hörgeräts (4), das einen Sensor (28), ein Mikrofon (16) und einen Hörer (22) aufweist. Anhand von mittels des Sensors (28) erstellten Messdaten (40) wird auf eine Atembeschwerde (52) eines Trägers (2) geschlossen und basierend hierauf ein Maß (62) für ein Risiko ermittelt. In Abhängigkeit des Maßes (62) wird eine dem Träger (2) helfende Tätigkeit (68) durchgeführt. Ferner betrifft die Erfindung ein Hörgerät (4).

## Beschreibung

Die Erfindung betrifft ein Verfahren zum Betrieb eines Hörgeräts und ein Hörgerät. Das Hörgerät weist jeweils einen Sensor, ein Mikrofon und einen Hörer auf. Bevorzugt ist das Hörgerät ein Hörhilfegerät.

Personen, die unter einer Verminderung des Hörvermögens leiden, verwenden üblicherweise ein Hörhilfegerät. Hierbei wird meist mittels eines Mikrofons, also eines elektromechanischen Schallwandlers, ein Umgebungsschall in ein elektrisches (Audio-/Schall-)Signal gewandelt, sodass das elektrische Signal erfasst wird. Die erfassten elektrischen Signale werden mittels einer Verstärkerschaltung bearbeitet und mittels eines weiteren elektromechanischen Wandlers in Form eines Hörers in den Gehörgang der Person eingeleitet. Meist erfolgt zudem eine Bearbeitung der erfassten Schallsignale, wofür üblicherweise ein Signalprozessor der Verstärkerschaltung verwendet wird. Hierbei ist die Verstärkung auf einen etwaigen Hörverlust des Hörgeräteträgers abgestimmt.

Atembeschwerden ("Dyspnoe") treten bei bis zu einem Viertel der Weltbevölkerung auf. Hierbei ist das Atmen für die jeweilige Person erschwert, sodass ein Komfort verringert ist. Aufgrund des erschwerten Atmens können dabei Angst- und Panikzustände hervorgerufen werden. Es können jedoch auch zusätzlich zu der subjektiv unangenehmen Erfahrung noch weitere Effekte auftreten, die zum Teil lebensbedrohliche sein können. Derartige Atem(not)beschwerden können akut auftreten, insbesondere in bestimmten Situationen, wie nach einer körperlichen Anstrengung. Sie können jedoch auch chronische sein. Atembeschwerden können als Symptom von Asthma, Stress, allergischen Reaktionen, einer Herzkrankheit oder sonstigen Krankheiten auftreten. In Abhängigkeit von dem jeweiligen Grund von aktuell auftretenden Atembeschwerden ist daher eine medizinische Versorgung der jeweiligen Person erforderlich, oder es sind von dieser bestimmte Tätigkeiten durchzuführen.

Der Erfindung liegt die Aufgabe zugrunde, ein besonders geeignetes Verfahren zum Betrieb eines Hörgeräts sowie ein besonders geeignetes Hörgerät anzugeben, wobei insbesondere ein Funktionsumfang und/oder eine Sicherheit für einen Träger erhöht ist.

Hinsichtlich des Verfahrens wird diese Aufgabe durch die Merkmale des Anspruchs 1 und hinsichtlich des Hörgeräts durch die Merkmale des Anspruchs 10 erfindungsgemäß gelöst. Vorteilhafte Weiterbildungen und Ausgestaltungen sind Gegenstand der jeweiligen Unteransprüche.

Das Verfahren dient dem Betrieb eines Hörgeräts. Beispielsweise ist das Hörgerät ein Kopfhörer oder umfasst einen Kopfhörer. Alternativ ist das Hörgerät ein Headset, True Wireless Headphone, Hearable, Personal Sound Amplifier. Besonders bevorzugt ist das Hörgerät jedoch ein Hörhilfegerät. Das Hörhilfegerät dient der Unterstützung einer unter einer Verminderung des Hörvermögens leidenden Person. Mit anderen Worten ist das Hörhilfegerät ein medizinisches Gerät, mittels dessen beispielsweise ein partieller Hörverlust ausgeglichen wird. Das Hörhilfegerät ist beispielsweise ein "Receiver-in-the-canal"-Hörhilfegerät (RIC; Ex-Hörer-Hörhilfegerät), ein Im-Ohr-Hörhilfegerät, wie ein "in-the-ear"-Hörhilfegerät, ein "in-the-canal"-Hörhilfegerät (ITC) oder ein "complete-in-canal"-Hörhilfegerät (CIC), eine Hörbrille, ein Taschenhörhilfegerät, ein Knochenleitungs-Hörhilfegerät oder ein Implantat. In einer weiteren Alternative ist das Hörhilfegerät ein Hinter-dem-Ohr-Hörhilfegerät ("Behind-the-Ear"-Hörhilfegerät), das hinter einer Ohrmuschel getragen wird.

Das Hörgerät ist vorgesehen und eingerichtet, am menschlichen Körper getragen zu werden. Mit anderen Worten umfasst das Hörgerät bevorzugt eine Haltevorrichtung, mittels deren eine Befestigung am menschlichen Körper möglich ist. Sofern es sich bei dem Hörgerät um ein Hörhilfegerät handelt, ist das Hörgerät vorgesehen und eingerichtet, beispielsweise hinter dem Ohr oder innerhalb eines Gehörgangs angeordnet zu werden. Insbesondere ist das Hörgerät kabellos und dafür vorgesehen und eingerichtet, zumindest teilweise in einen Gehörgang eingeführt zu werden. Besonders bevorzugt umfasst das Hörgerät einen Energiespeicher, mittels dessen eine Energieversorgung bereitgestellt ist.

Das Hörgerät umfasst ferner ein Mikrofon, das dem Erfassen von Schall dient. Insbesondere wird bei Betrieb mittels des Mikrofons ein Umgebungsschall erfasst, oder zumindest ein Teil hiervon. Bei dem Mikrofon handelt es sich insbesondere um einen elektromechanischen Schallwandler. Das Mikrofon weist beispielsweise lediglich eine einzige Mikrofoneinheit oder mehrere Mikrofoneinheiten auf, die miteinander wechselwirken. Jede der Mikrofoneinheiten weist zweckmäßigerweise eine Membran auf, die anhand von Schallwellen in Schwingungen versetzt wird, wobei die Schwingungen mittels eines entsprechenden Aufnahmegeräts, wie eines Magneten, der in einer Spule bewegt wird, in ein elektrisches Signal gewandelt wird. Somit ist es möglich, mittels der jeweiligen Mikrofoneinheit ein Audiosignal zu erfassen, das auf dem auf die Mikrofoneinheit auftreffendem Schall basiert. Die Mikrofoneinheiten sind insbesondere unidirektional ausgestaltet. Das Mikrofon ist zweckmäßigerweise zumindest teilweise innerhalb eines Gehäuses des Hörgeräts angeordnet und somit zumindest teilweise geschützt.

Ferner weist das Hörgerät einen Hörer zum Ausgeben eines Ausgabesignals auf. Das Ausgabesignal ist hierbei insbesondere ein elektrisches Signal. Der Hörer ist ein elektromechanischer Schallwandler, vorzugsweise ein Lautsprecher. Je nach Ausgestaltung des Hörgeräts ist im bestimmungsgemäßen Zustand der Hörer zumindest teilweise innerhalb eines Gehörgangs eines Trägers des Hörgeräts, also einer Person, angeordnet oder zumindest akustisch mit diesem verbunden. Das Hörgerät dient insbesondere hauptsächlich dem Ausgeben des Ausgabesignals mittels des Hörers, wobei sprechende Schall erstellt wird. Mit anderen Worten ist die Hauptfunktion des Hörgeräts das Ausgeben des Ausgabesignals. Das Ausgabesignal ist dabei insbesondere zumindest teilweise in Abhängigkeit des mittels des Mikrofons erfassten Schalls erstellt. Alternativ ist das Ausgabesignal in Abhängigkeit eines übertragenen Datensignals erstellt, oder das Datensignal wird hierfür verwendet. Mit anderen Worten wird das Ausgabesignal insbesondere anhand eines Streamings-Vorgangs erstellt, oder es handelt sich hierbei um eine Wiedergabe eines bestimmten Samples.

Das Hörgerät weist ferner einen Sensor auf, mittels dessen bei Betrieb Messdaten erstellt werden. Der Sensor ist hierbei beispielsweise mittels einer einzigen Einheit gebildet oder umfasst unterschiedliche Einheiten. Somit ist es beispielsweise möglich, mittels des Sensors unterschiedliche Signale zum Erfassen, die insbesondere auf unterschiedlichen physikalischen Gegebenheiten basieren, oder die sich an unterschiedlichen Orten ergeben, wobei an jedem dieser Orte eine entsprechende Einheit des Sensors angeordnet ist. Beispielsweise ist der Sensor mittels des Mikrofons gebildet oder umfasst dieses. Alternativ hierzu sind der Sensor und das Mikrofon zueinander separate Bauteile des Hörgeräts. Geeigneterweise korrespondieren die Messdaten zu dem Signal, das mittels des Sensors erfasst wird. Die Messdaten sind insbesondere elektrische Signale. Ausführungsbeispiele für Sensoren, die diese Messdaten erfassen, erzeugen oder umformen sind: ein Elektroenzephalographie-Sensor, ein Elektrokardiographie-Sensor, ein Photoplethysmographie-Sensor, ein Nahinfrarotspektroskopie-Sensor, ein Elektrookulographie-Sensor, ein Elektromygraphie-Sensor, ein Sensor für eine Muskelspannung, ein Pupillometrie-Sensor, ein Mikroexpression-Sensor, ein Sensor zur Erfassung von Atemparameternmung, ein Atemtiefe-Sensor, ein Atemfrequenz-Sensor, ein Herzfrequenz-Sensor, ein Sensor für die Herzratenvariabilität, ein Blutdruck-Sensor, ein Sensor für eine kontraktile Eigenschaft des Herzens, ein Mikrofon, eine Inertiale Messeinheit, ein Vibrometer, ein Cortisol-Sensor, ein Schweiß-Sensor, ein Sensor für den Hautleitwert, ein Temperatursensor, ein Sensor für die Sauerstoffsättigung des Blutes, ein Bioimpedanz-Sensor, ein auskultatorischer Sensor, ein Kapnometrie-Sensor

Gemäß dem Verfahren wird anhand von mittels des Sensors erstellten Messdaten auf eine Atembeschwerde des Trägers des Hörgeräts geschlossen. Die Messdaten sind dabei insbesondere derart, dass anhand dieser auf eine Atembeschwerde des Trägers geschlossen werden kann, und der Sensor ist entsprechend ausgebildet. Zusammenfassend werden zunächst die Messdaten erstellt und diese analysiert und/oder ausgewertet. Anhand der Messdaten wird abgeleitet, ob der Träger aktuell, oder zumindest zum Zeitpunkt des zu den Messdaten korrespondierenden Signals unter Atembeschwerden litt. Basierend hierauf wird ein Maß für ein Risiko ermittelt. Mit anderen Worten wird insbesondere anhand des Vorhandenseins der Atembeschwerden ein Maß für ein Risiko für den Träger abgeleitet. Beispielsweise ist das Maß lediglich binär, und der eine Zustand wird verwendet, wenn die Atembeschwerden vorliegen, und ansonsten der andere Zustand. Alternativ hierzu weist beispielsweise das Maß mehrere Stufen auf, wobei für die Zuordnung des jeweiligen Werts des Maßes lediglich das Vorhandensein der Atembeschwerden herangezogen wird oder besonders bevorzugt noch weitere Parameter. Beispielsweise wird auch eine Schwere der Atembeschwerden für das Ermitteln des Maßes für das Risiko verwendet. Insbesondere wird hierfür ein Schwellwertvergleich durchgeführt.

In Abhängigkeit des Maßes wird eine den Träger helfende Tätigkeit durchgeführt. Die helfende Tätigkeit ist hierbei derart, dass der Träger in seiner aktuellen Situation, also bei Vorliegen der Atembeschwerden, unterstützt wird, sodass diesem geholfen wird. Hierbei betrifft die Tätigkeit die Atembeschwerden, und diese sorgt beispielsweise dafür, dass die Atembeschwerden verringert werden, und/oder dass der Träger des Hörgeräts beim Bewältigen der Situation, in der die Atembeschwerden unterstützt wird.

Beispielsweise wird die Tätigkeit lediglich durchgeführt, wenn das Maß für das Risiko einen bestimmten Schwellwerts überschreitet. Alternativ liegen beispielsweise liegen unterschiedliche Tätigkeiten zur Auswahl vor, wobei in Abhängigkeit des Maßes die jeweilige Tätigkeit durchgeführt wird. Mit anderen Worten werden bei unterschiedlichen Maßen für das Risiko unterschiedliche Tätigkeiten durchgeführt.

Aufgrund des Verfahrens wird somit eine den Träger des Hörgeräts helfende Tätigkeit durchgeführt, wenn dieser unter Atembeschwerden leidet, die insbesondere ein vergleichsweise großes Risiko darstellen. Somit wird vorzugsweise das Risiko für den Träger des Hörgeräts reduziert. Zumindest ist für den Träger des Hörgeräts, der auch als Nutzer bezeichnet wird, ein Komfort erhöht. Auch ist auf diese Weise ein Funktionsumfang des Hörgeräts vergrößert. Atemnot kann beispielsweise ein Symptom sein von einer chronisch obstruktiven Lungenerkrankung (weltweite Prävalenz etwa 7-19%), einem Asthma bronchiale (weltweite Prävalenz etwa 5%), körperlicher Belastung wie Sport oder erschöpfendem Husten, psychologischem Stress, allergischen Reaktionen, Krankheiten des Herzens, einer Herzinsuffizienz, einem akutem Koronarsyndrom mit retrosternalem Unwohlsein und Atembeschwerden, COVID-19 und weiteren die Lunge betreffenden pathologischen Zuständen wie einer Pneumonie, einem Pneumothorax oder einer Lungenembolie. Eine akute Atembeschwerde kann zum Ersticken führen. Ebenso können Begleitsymptome von einer eine Atembeschwerde hervorrufende Krankheit die Gesundheit schädigen, wenn eine Behandlung ausbleibt oder zu spät stattfindet. Atembeschwerden sollten registriert werden, da sie in anderen Situationen eine schwerwiegendere Ausprägung haben können, beispielsweise bei erhöhter körperlicher Belastung oder veränderten Umweltbedingungen.

Sobald auf die Atembeschwerden geschlossen wurde, wird dies vorzugsweise in einem Speicher hinterlegt, sodass ein Protokoll hierüber vorhanden ist. Der Speicher ist beispielsweise ein Bestandteil des Hörgeräts oder eines damit kommunikativ verbundenen externen Geräts. Insbesondere wird zusätzlich auch der jeweilige Zeitpunkt abgespeichert. Dies erfolgt insbesondere unabhängig von dem Maß für das Risiko, sodass stets bei vorhandenen Atembeschwerden ein entsprechender Eintrag in dem Protokoll erfolgt. Vorzugsweise wird zusätzlich auch das Maß für das Risiko abgespeichert und/oder die etwaige Tätigkeit, die durchgeführt wird. In einer Weiterbildung wird beispielsweise auch eine etwaige akustische Klassifikation und/oder sonstige Betriebsdaten abgespeichert. Alternativ hierzu erfolgt das Abspeichern lediglich, wenn das Maß für das Risiko einen bestimmten Wert überschreitet oder entspricht. Auf diese Weise ist ein Platzbedarf für das Protokoll verringert. Aufgrund des Protokolls ist eine Ermittlung eines Gesundheitszustands des Trägers in einem separaten Diagnoseverfahren ermöglicht.

Besonders bevorzugt wird anhand der Messdaten eine aktuelle Körperhaltung des Trägers ermittelt. Hieraus wird nachfolgend auf die Atembeschwerde geschlossen. Insbesondere wird auf die Atembeschwerden geschlossen, wenn eine bestimmte Körperhaltung von dem Träger eingenommen wurde. Geeigneterweise wird auf die Atembeschwerde geschlossen, wenn bei der Körperhaltung eine Vorspannung von Muskeln erfolgt, die zum Atmen erforderlich sind oder dieses zumindest unterstützen. Aufgrund der bestimmten Körperhaltung erfolgt insbesondere ein Druck im Bauchbereich und/oder im Rippenbereich des Trägers. Vorzugsweise wird dabei auf die Atembeschwerde geschlossen, wenn die jeweilige Körperhaltung mindestens eine bestimmte Zeitspanne angehalten wird. Die Zeitspanne ist dabei vorzugsweise 5 Sekunden, 10 Sekunden oder 30 Sekunden.

Manche Körperhaltungen habe eine atemerleichternde Wirkung, indem sie die Atemhilfsmuskeln in einem günstigen Maß vorspannen. Atemhilfsmuskeln unterstützen beispielsweise die Elevation oder Depression von Rippen oder die Bauchpresse. Manche Atemhilfsmuskeln unterstützen das Einatmen, manche unterstützen das Ausatmen. Abhängig von der Körperhaltung sind unterschiedliche Muskeln zu einem unterschiedlichen Maß angespannt oder vorgespannt. Hals-, Nacken-, Schulter-, Rumpf-, Brust-, Bauch- oder Rückenmuskulatur, welche die Atmung unterstützen kann, kann ausgewählt sein aus der Gruppe von Muskeln: Musculus sternocleidomastoideus, Musculi scaleni, Musculus scalenus anterior, Musculus scalenus medius, Musculus scalenus posterior, Musculus latissimus dorsi, Musculus transversus thoracis, Musculi pectorales, Musculus pectoralis major, Musculus pectoralis minor, Musculi serrati, Musculus serratus posterior superior, Musculus serratus posterior inferior, Musculus serratus anterior, Musculus rectus abdominis, Musculus transversus abdominis, Musculus obliquus externus abdominis, Musculus obliquus internus abdominis, Musculus quadratus lumborum.

Als die bestimmte Körperhaltung oder eine der bestimmten Körperhaltungen wird beispielsweise der sogenannte Kutschersitz herangezogen. Bei diesem sitzt der Träger, wobei der Oberkörper nach vorne geneigt und der Kopf nach unten gerichtet ist. Auch sind die Armen auf den Oberschenkeln abgestützt. Vorzugsweise ist dabei der Sensor ein Beschleunigungssensor und/oder Positionssensor oder umfasst zumindest diese. Insbesondere korrespondieren die Messdaten, anhand derer auf den Kutschersitz geschlossen wird, zu einer kreisförmigen Vorwärtsbewegung des Kopfes, die abrupt beendet wird, und/oder zu einer Rotation um eine Transversalachse, beispielsweise auf Höhe des Beckens des Trägers.

Eine andere derartige (bestimmte) Körperhaltung ist in der sogenannte Paschasitz, bei dem der Träger mit einem durchgestreckten Rücken und leichten geneigtem Kopf sitzt. Dabei ist die Zeitdauer, die diese Körperhaltung eingehalten wird, größer als länger als bei einer Dehnbewegung ist, und insbesondere länger als 10 Sekunden. Auch hier ist bzw. umfasst der Sensor beispielsweise ein Beschleunigungs- und/oder Positionssensor, und die Messdaten korrespondieren zu einem Strecken der Wirbelsäule.

Eine andere derartige (bestimmte) Körperhaltung ist ein vorgebeugtes Stehen, wobei die Arme abgestützt sind, beispielsweise auf einem Stuhl oder an einer Wand. Insbesondere korrespondieren in diesem Fall die Messdaten zu einer kreisbogenförmigen Bewegung des Kopfes, die abrupt beendet wird. Eine andere derartige (bestimmte) Körperhaltung ist ein nachhinten gelehnter Stand, wobei der Rücken abgestützt ist, beispielsweise an einer Wand, und wobei der Kopf leicht nach unten angewinkelt ist. Insbesondere korrespondieren in diesem Fall die Messdaten ebenfalls zu einer kreisbogenförmigen Bewegung des Kopfes.

Die beiden oben genannten bestimmten Körperhaltungen, bei denen sich der Kopf des Trägers im Bereich einer Wand und somit auch das Mikrofon in vergleichsweise geringer Entfernung von der Wand befindet, sind auch anhand der mittels des Mikrofons erstellten elektrischen Signale ermittelbar, da der damit erfasste Schall bestimmte Eigenschaften aufweist. Insbesondere wird hierbei mittels des Mikrofons zusätzlichen die ermittelte Körperhaltung bestätigt, oder die mittels des Mikrofons erstellten Signale werden als Messdaten zumindest teilweise herangezogen, sodass das Mikrofon zumindest teilweise den Sensor bildet.

Eine andere derartige (bestimmte) Körperhaltung ist beispielsweise die Hocke, wobei die Hände auf den Knien oder den Oberschenkeln abgestützt sind. Hierbei ist der Kopf in Richtung des Bodens gerichtet.

Eine der oben genannten (bestimmten) Körperhaltungen wird von dem Träger zum Beispiel teilweise unbewusst eingenommen, um das Atmen zu erleichtern, wobei dies unabhängig von dem Alter und Geschlecht des Trägers ist. Somit liegen die Atembeschwerden vor, wenn der Träger eine der bestimmten Körperhaltungen einnimmt, was mittels des Sensors ermittelt wird. Aufgrund der Ermittlung der Atembeschwerden anhand der Körperhaltung ist kein direktes Erfassen von Vitalfunktionen des Trägers erforderlich, weswegen ein Hardwarebedarf reduziert ist. Auch ist dies unabhängig von dem Alter und Geschlecht des Trägers, weswegen es nicht erforderlich ist, eine spezielle Einstellung des Hörgeräts vorzunehmen. Somit ist das Hörgerät von unterschiedlichen Personen Trägern verwendbar.

Beispielsweise wird die ermittelte Körperhaltung auch beim Ermitteln des Maßes für das Risiko verwendet. So werden beispielsweise einige der bestimmten Körperhaltungen bei einem höheren Risiko und andere bei einem niedrigeren Risiko verwendet.

Alternativ oder in Kombination hierzu wird anhand von mittels des Mikrofons erstellten weiteren Messdaten ein die Atmung des Trägers charakterisierender Wert ermittelt. Beispielsweise wird als charakterisierender Wert die Atemfrequenz oder eine Tiefe des Atmens, die zu einem eingeatmeten Luftvolumen korrespondiert, verwendet, oder der charakterisierende Wert umfasst zumindest eines hiervon. Alternativ oder in Kombination hierzu umfasst der charakterisierende Wert beispielsweise das Vorhandensein und oder eine Stärke eines Rauschens oder Rasselns beim Atmen, zum Beispiel beim Ein- oder bevorzugt beim Ausatmen. Ein derartiges Rauschen tritt insbesondere auf, wenn die Lippen des Trägers zusammengepresst sind, sodass ein Gegendruck beim Ausatmen entsteht, das Atmen erleichtert. Dies erfolgt insbesondere dadurch, dass die Bronchen geweitet werden.

Der charakterisierende Wert wird zum Schließen auf die Atembeschwerden verwendet. Da das Mikrofon bereits vorhanden ist, sind somit keine zusätzlichen Bauteile erforderlich, weswegen Herstellungskosten des Hörgeräts reduziert sind. Beispielsweise stellte dabei das Mikrofon den Sensor dar, und die weiteren Messdaten entsprechend den Messdaten, sodass Herstellungskosten weiter reduziert sind. Besonders bevorzugt jedoch ist der Sensor und das Mikrofon vorhanden, die insbesondere zueinander separate Bauteile darstellen. Somit ist die Genauigkeit beim Schließen auf die Atembeschwerden verbessert.

Beispielsweise wird anhand eines Algorithmus basierend auf den Messdaten auf die Atembeschwerden geschlossen. Der Algorithmus ist beispielsweise fest hinterlegt oder an den jeweiligen Träger angepasst. Besonders bevorzugt jedoch wird zum Schließen auf die Atembeschwerden ein neuronales Netz verwendet. Dieses wird insbesondere während des Tragens des Hörgeräts von dem Träger angelernt, wobei das neuronale Netz beispielsweise bereits vor dem ersten Gebrauch zumindest rudimentär angelernt ist. Somit wird mit zunehmender Tragedauer eine Genauigkeit beim Ermitteln der Atembeschwerden erhöht und somit das Hörgerät auf den jeweiligen Träger angepasst. Auch ist auf diese Weise kein genaues Wissen um den Körperbau des Trägers bei Herstellung des Hörgeräts erforderlich.

Alternativ oder in Kombination hierzu wird das neuronale Netz zum Vorhersagen von Atembeschwerden herangezogen, sodass das Maß für das Risiko insbesondere ein Maß für das Eintreten der Atembeschwerden darstellt. Mittels Durchführens der helfenden Tätigkeit wird hierbei ein tatsächliches Eintreten der Atembeschwerden verhindert oder deren Stärke zumindest verringert. Somit ist ein Komfort für den Träger des Hörgeräts weiter erhöht.

Beispielsweise erfolgt das Ermitteln des Maßes für das Risiko unabhängig von weiteren Parametern und zum Beispiel lediglich anhand der Messdaten. Besonders bevorzugt jedoch werden weitere Einflussfaktoren/Parameter berücksichtigt. Vorzugsweise wird beim Ermitteln des Maßes für das Risiko eine ermittelte körperliche Aktivität des Trägers berücksichtigt. Mit anderen Worten wird ermittelt, dass der Träger vor dem Auftreten oder beim Auftreten der Atembeschwerden eine körperliche Aktivität durchführt oder nicht. Beispielsweise werden zum Ermitteln der körperlichen Aktivität die gleichen Messdaten oder unterschiedliche Messdaten verwendet, die mittels des oder eines anderen Sensors zeitlich vorhergehend erstellt wurden. Auf diese Weise ist beispielsweise ableitbar, ob die Atembeschwerden aufgrund der körperlichen Aktivität auftreten, beispielsweise einer sportlichen Aktivität, und/oder einer vergleichsweise starken körperlichen Belastung, wie zum Beispiel Treppensteigen. Da in diesem Fall nach einer vergleichsweise kurzen Zeitspanne die Atembeschwerden nachlassen, ist das Maß für das Risiko verringert. Mit anderen Worten wird ein verringertes Maß für das Risiko verwendet, wenn vor Auftreten der Atembeschwerden eine körperliche Aktivität von dem Träger durchgeführt wurde. Falls hingegen die Atembeschwerden auftreten, wenn zeitlich vorhergehend im Wesentlichen keine körperliche Aktivität stattfand, ist das Maß für das Risiko erhöht.

Besonders bevorzugt werden gemäß dem Verfahren von einem externen Sensor externe Daten empfangen, die den körperlichen Zustand des Trägers betreffen. Der externe Sensor ist zum Beispiel mobil, tragbar, an der Kleidung befestigbar, implantierbar und/oder ein Terminal. Insbesondere werden die externen Daten mittels des externen Sensors erstellt und sind beispielsweise ebenfalls Messdaten, oder die externen Daten sind bereits zumindest teilweise ausgewertete Messdaten. Als ein derartiger externer Sensor wird beispielsweise ein Elektrokardiographie-Sensor (EKG), ein Auskultation-Sensor, ein Photoplethysmographie-Sensor oder ein Ballistokardiographie-Sensor verwendet. Alternativ oder in Kombination hierzu betreffend oder entsprechen die externen Daten eine Pulsfrequenz, eine Änderung des Pulses, einen Blutdruck, einen Sauerstoffgehalt des Blutes, eine Körpertemperatur, eine Hauttemperatur eine Atemfrequenz oder eine Atemtiefe. In einer weiteren Alternative betreffen die externen Daten eine Schweißabsonderung des Trägers, die insbesondere anhand von einer elektrodermalen Aktivität, beispielsweise eines Sensors für den Hautleitwert, und/oder mittels eines VCSEL-Lasers und eines Photodetektors erstellt werden. Die externen Daten werden beim Ermitteln des Maßes für das Risiko verwendet. So wird beispielsweise bei einem erhöhten Blutdruck und/oder erhöhter Schweißabsonderung ein höheres Maß für das Risiko verwendet, als bei einem niedrigeren Blutdruck/geringer Schweißabsonderung. Alternativ oder in Kombination hierzu werden externen Daten auch zum Schließen auf die Atembeschwerden verwendet. Ausführungsbeispiele für einen derartigen externen Sensoren sind beispielsweise: ein Elektroenzephalographie-Sensor, ein Elektrokardiographie-Sensor, ein Photoplethysmographie-Sensor, ein Nahinfrarotspektroskopie-Sensor, ein Elektrookulographie-Sensor, ein Elektromygraphie-Sensor, ein Sensor für eine Muskelspannung, ein Pupillometrie-Sensor, ein Mikroexpression-Sensor, ein Sensor zur Erfassung von Atemparameternmung, ein Atemtiefe-Sensor, ein Atemfrequenz-Sensor, ein Herzfrequenz-Sensor, ein Sensor für die Herzratenvariabilität, ein Blutdruck-Sensor, ein Sensor für eine kontraktile Eigenschaft des Herzens, ein Mikrofon, eine Inertiale Messeinheit, ein Vibrometer, ein Cortisol-Sensor, ein Schweiß-Sensor, ein Sensor für den Hautleitwert, ein Temperatursensor, ein Sensor für die Sauerstoffsättigung des Blutes, ein Bioimpedanz-Sensor, ein auskultatorischer Sensor, ein Kapnometrie-Sensor

Vorzugsweise ist der externe Sensor ein Bestandteil eines externen Geräts, das von dem Träger getragen wird, und das beispielsweise signaltechnisch mit dem Hörgerät gekoppelt ist. Insbesondere weist hierfür das Hörgerät eine entsprechende Vorrichtung auf. Zweckmäßigerweise erfolgt die signaltechnische Kopplung mittels Funk, also kabellos. Alternativ hierzu sind diese kabelgebundenen signaltechnisch gekoppelt. Das externe Gerät ist insbesondere ein Fitnessarmband oder ein Mobiltelefon, wie ein Smartphone. Die Erfindung betrifft auch ein System mit dem Hörgerät und dem externen Gerät, das den externen Sensor aufweist.

In einer Weiterbildung des Verfahrens wird eine aktuelle Umgebungssituation ermittelt, wobei dies insbesondere dann erfolgt, nachdem auf die Atembeschwerden geschlossen wurde. Alternativ oder in Kombination hierzu wird die Umgebungssituation bereits für den Betrieb des Hörgeräts verwendet, insbesondere für den Betrieb des Hörers. Bevorzugt wird in Abhängigkeit der aktuellen Umgebungssituation die helfende Tätigkeit durchgeführt. So wird insbesondere bei einem mittleren Maß für das Risiko und einer Umgebungssituation, bei der weitere Personen anwesend sind, eine Bitte, insbesondere eine Aufforderung in Form einer Nachricht, an diese abgegeben, dem Träger des Hörgeräts zu helfen. Hierfür wird insbesondere ein sprechendes Schallsignal ausgegeben, zum Beispiel mittels des Hörers oder eines weiteren Ausgabegeräts. Alternativ wird beispielsweise ein Alarm auf einem Smartphone oder einem anderen Gerät veranlasst. In einer weiteren Alternative wird beispielsweise ein weiterer Hörgeräteträger alarmiert, wobei mittels dessen Hörers die Bitte ausgegeben wird. Dabei wird zwischen den beiden Hörgeräten die Bitte ausgetauscht, vorzugsweise über ein Peer2Peer Netzwerk.

Falls die aktuelle Umgebungssituation zum Beispiel zu einem Theater- oder Opernbesuch entspricht, erfolgt die Ausgabe dahingegen lediglich bei einem vergleichsweise großen Maß für das Risiko ausgegeben. Dahingegen wird bei einer Teilnahme am Straßenverkehr die Ausgabe bereits bei einem verringerten Maß durchgeführt, sodass weitere Verkehrsteilnehmer auf den eingeschränkten Gesundheitszustand des Trägers hingewiesen werden, auch wenn zum Beispiel eine Hilfe von diesen nicht erforderlich ist. Die Ausgabe erfolgt dabei beispielsweise auf mobilen Geräten von Personen in der Nähe und/oder auf einem terminal Gerät, beispielsweise einer großen Anzeigetafel, die für eine Gruppe von Personen in der Nähe zu sehen ist und auf die Notsituation und sie charakterisierende Parameter (z.B. Art des Problems, Ort, Richtung oder Entfernung des Trägers) aufmerksam macht.

Alternativ oder in Kombination hierzu wird auch anhand der aktuellen Umgebungssituation das Maß für das Risiko ermittelt. So wird beispielsweise bei Einnahme einer Körperhaltung, anhand derer auf die Atembeschwerden geschlossen wird oder werden kann, und die vergleichsweise unnatürlich wirkt, ein vergleichsweise hohes Risiko angenommen, wenn anhand der aktuellen Umgebungssituation darauf geschlossen wird, dass sich der Träger in der Öffentlichkeit befindet. Wenn trotz der Öffentlichkeit diese Körperhaltung eingenommen wird, wird davon ausgegangen, dass die Atembeschwerden vergleichsweise stark sind. Wenn diese Körperhaltung hingegen in privaten Räumen, also wenn insbesondere keine weitere Person anwesend sind, verwendet wird, wird ein vermindertes Maß für das Risiko verwendet.

Beispielsweise umfasst die helfende Tätigkeit die Ausgabe einer Anweisung zur Einnahme einer die Atmung verstärkt unterstützenden Körperhaltung. Vorzugsweise wird hierbei die Anweisung mittels des Hörers ausgegeben, sodass die Anweisung zumindest für den Träger des Hörgeräts wahrnehmbar ist. Insbesondere erfolgt das Ausgeben der Anweisung derart, dass diese im Wesentlichen lediglich von dem Träger des Hörgeräts wahrnehmbar ist. Wenn der Träger des Hörgeräts die die Atmung verstärkt unterstützende Körperhaltung einnimmt, werden bestimmte Muskeln des Trägers vorgespannt, sodass das Atmen für den Träger erleichtert ist. Insbesondere wird hierfür eine der oben genannten bestimmten Körperhaltungen verwendet. Sofern bereits anhand der Körperhaltung auf das Vorliegen der Atembeschwerden geschlossen wird, wird insbesondere in der Anweisung eine andere der bestimmten Körperhaltungen angegeben, bei der die Atmung verstärkt unterstützt wird, also die der die Atembeschwerden stärker gelindert werden als in der aktuellen Körperhaltung des Trägers.

Alternativ oder in Kombination hierzu umfasst die helfende Tätigkeit das Aussenden einer Notfallmeldung mittels einer Kommunikationsvorrichtung des Hörgeräts. Vorzugsweise wird diese helfende Tätigkeit lediglich bei einem vergleichsweise hohen Maß für das Risiko durchgeführt. Die Notfallmeldung ist insbesondere an eine Rettungsleitstelle, einen Rettungsdienst und/oder einen Notarzt gerichtet. Auf diese Weise ist eine Reaktionszeit zum zur medizinischen Versorgung des Trägers durch diese reduziert. Die Kommunikationsvorrichtung ist insbesondere gemäß einem Mobilfunk- und/oder WLAN-Standard betrieben, und die Notfallmeldung wird direkt von dem Hörgerät zu dem gewünschten Empfänger der Notfallmeldung gesandt. Alternativ hierzu ist die Kommunikationsvorrichtung gemäß einem Bluetooth-Standard betrieben und zum Beispiel an das etwaige externe Gerät gerichtet, das zum Beispiel ein Mobiltelefon ist. Von diesen wird nachfolgend die Notfallmeldung entsprechend eines Mobilfunk- und/oder WLAN-Standard weiter übertragen. Auf diese Weise ist ein Energiebedarf für den Betrieb des Hörgeräts verringert, wobei dennoch die Notfallmeldung sicher übertragen wird.

Das Hörgerät weist ein Mikrofon, und einen Hörer auf, zwischen die insbesondere eine Signalverarbeitungseinheit signaltechnisch geschaltet ist. Mittels dieser ist insbesondere ein Signalpfad gebildet, und das Mikrofon dient vorzugsweise dem Erfassen von Schall und der Hörer geeigneterweise dem Ausgeben von Schall. Beispielsweise ist das Hörgerät ein Kopfhörer oder umfasst einen Kopfhörer. Hierbei ist das Hörgerät beispielsweise als ein sogenanntes Headset ausgestaltet. Besonders bevorzugt ist das Hörgerät jedoch ein Hörhilfegerät. Das Hörhilfegerät dient der Unterstützung einer unter einer Verminderung des Hörvermögens leidenden Person. Mit anderen Worten ist das Hörhilfegerät ein medizinisches Gerät, mittels dessen beispielsweise ein partieller Hörverlust ausgeglichen wird. Das Hörhilfegerät ist beispielsweise ein "receiver-in-the-canal"-Hörhilfegerät (RIC; Ex-Hörer-Hörhilfegerät), ein Im-Ohr-Hörhilfegerät, wie ein "in-the-ear"-Hörhilfegerät, ein "in-the-canal"-Hörhilfegerät (ITC), ein "complete-in-canal"-Hörhilfegerät (CIC) oder ein "invisible in the canal"-Hörhilfegerät (IIC), eine Hörbrille oder ein Taschenhörhilfegerät. Besonders bevorzugt ist das Hörhilfegerät ein Hinter-dem-Ohr-Hörhilfegerät ("Behind-the-Ear"-Hörhilfegerät), das hinter einer Ohrmuschel getragen wird. Ferner weist das Hörgerät einen Sensor zum Erstellen von Messdaten auf. Insbesondere ist hierbei anhand der Messdaten das Schließen auf Atembeschwerden des Trägers ermöglicht. Hierfür ist der Sensor geeignet aufgebaut und/oder ausgestaltet.

Das Hörgerät ist gemäß einem Verfahren betrieben, bei dem anhand von mittels des Sensors erstellten Messdaten auf eine Atembeschwerde eines Trägers geschlossen und basierend hierauf ein Maß für ein Risiko ermittelt wird. In Abhängigkeit des Maßes wird eine dem Träger helfende Tätigkeit durchgeführt wird, sodass dem Träger insbesondere direkt oder als Konsequenz befolgter Anweisungen geholfen wird. Beispielsweise erfolgt das Schließen auf die Atembeschwerden, das Ermitteln des Maßes für das Risiko und/oder das Durchführen der Tätigkeit mittels der Signalverarbeitungseinheit. Mit anderen Worten ist die Signalverarbeitungseinheit geeignet, insbesondere vorgesehen und eingerichtet, das Verfahren zumindest teilweise durchzuführen.

Das Hörgerät umfasst zweckmäßigerweise einen Signalprozessor, der geeigneterweise die Signalverarbeitungseinheit bildet oder zumindest ein Bestandteil hiervon ist. Der Signalprozessor ist beispielsweise ein digitaler Signalprozessor (DSP) oder mittels analoger Komponenten realisiert. Mittels des Signalprozessors erfolgt insbesondere auch eine Anpassung des mittels des Mikrofons erstellten Signals, vorzugsweise in Abhängigkeit eines etwaigen Hörverlust eines Trägers des Hörgeräts. Zweckmäßigerweise ist zwischen dem Mikrofon und der Signalverarbeitungseinheit, beispielsweise den Signalprozessor, ein A/D-Wandler angeordnet, sofern der Signalprozessor als digitaler Signalprozessor ausgestaltet ist. Der Signalprozessor ist insbesondere in Abhängigkeit eines Parametersatzes eingestellt. Mittels des Parametersatzes wird dabei eine Verstärkung in unterschiedlichen Frequenzbereichen vorgegeben, sodass das mittels des Mikrofons erstellte Signal entsprechend bestimmter Vorgaben bearbeitet wird, insbesondere in Abhängigkeit eines Hörverlust des Trägers des Hörgeräts. Besonders bevorzugt umfasst das Hörgerät zusätzlich einen Verstärker, oder der Verstärker ist mittels des Signalprozessors zumindest teilweise gebildet. Beispielsweise ist der Verstärker signaltechnisch dem Signalprozessor vor- oder nachgeschaltet.

Die im Zusammenhang mit dem Verfahren beschriebenen Weiterbildungen und Vorteile sind sinngemäß auch auf das Hörgerät zu übertragen und umgekehrt.

Nachfolgend wird ein Ausführungsbeispiel der Erfindung anhand einer Zeichnung näher erläutert. Darin zeigen:
- Fig. 1: schematisch einen Träger eines Hörgeräts,
- Fig. 2: schematisch das Hörgerät
- Fig. 3: ein Verfahren zum Betrieb des Hörgeräts, und
- Fig. 4-9: schematisch unterschiedliche, die Atmung unterstützende Körperhaltungen.

Einander entsprechende Teile sind in allen Figuren mit den gleichen Bezugszeichen versehen.

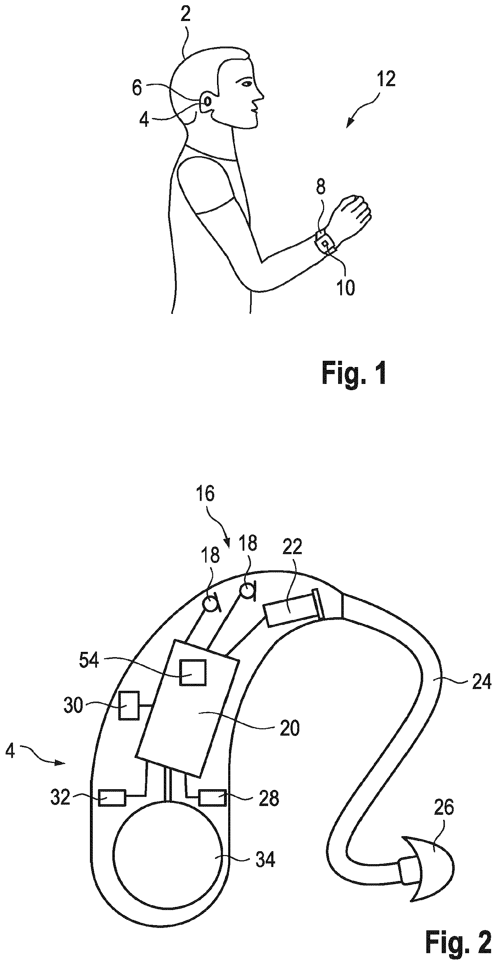

In Fig. 1 ist ein Träger 2 eines Hörgeräts 4 in Form eines Hörhilfegeräts gezeigt, das vorgesehen und eingerichtet ist, hinter einem Ohr 6 des Trägers 2 (Benutzer, Hörgeräteträger, Nutzer) getragen zu werden. Mit anderen Worten handelt es sich um ein Hinter-dem-Ohr-Hörhilfegerät ("Behind-the-Ear" - Hörhilfegerät). Der Träger 2 ferner ein externes Gerät 8 in Form eines Fitnessarmbands, das einen externen Sensor 10 aufweist. Mittels des externen Sensors 10 ist es möglich, eine Pulsfrequenz des Trägers 2 zu messen. Das externe Gerät 8 umfasst zudem einen Kommunikationsbaustein, der gemäß einem Mobilfunk-Standard betrieben ist. Somit ist es für den Träger 2 möglich, mittels des externen Geräts 8 zu telefonieren und/oder Daten zu empfangen. Auch ist der Kommunikationsbaustein zusätzlich zur Herstellung einer Bluetooth-Verbindung ertüchtigt. Im bestimmungsgemäßen Gebrauch sind das externe Gerät 8 und das Hörgerät 4 mittels einer Bluetooth-Verbindung signaltechnisch verbunden, sodass ein System 12 gebildet ist, das das externe Gerät 8 und das Hörgerät 4 umfasst.

Das Hörgerät 4 ist schematisch in Figur 2 dargestellt. Das Hörgerät 4 umfasst ein Gehäuse 14, das aus einem Kunststoff gefertigt ist. Innerhalb des Gehäuses 14 ist ein Mikrofon 16 mit zwei Mikrofoneinheiten 18 in Form von jeweils elektromechanischen Schallwandlern angeordnet, die omnidirektional ausgestaltet sind. Indem ein zeitlicher Versatz zwischen den mittels der omnidirektionalen Mikrofoneinheiten 18 erfassten akustischen Signalen verändert wird, ist es ermöglicht, eine Richtcharakteristik des Mikrofons 16 zu verändern, sodass ein Richtmikrofon realisiert ist. Die beiden Mikrofoneinheiten 18 sind mit einer Signalverarbeitungseinheit 20 signaltechnisch gekoppelt, die eine nicht näher gezeigte Verstärkerschaltung und einen Signalprozessor umfasst. Die Signalverarbeitungseinheit 20 ist ferner mittels Schaltungselementen gebildet, wie zum Beispiel elektrischen und/oder elektronischen Bauteilen. Der Signalprozessor ist ein digitaler Signalprozessor (DSP) und über einen nicht näher dargestellten A/D-Wandler signaltechnisch mit den Mikrofoneinheiten 18 verbunden.

Mit der Signalverarbeitungseinheit 20 ist ein Hörer 22 signaltechnisch gekoppelt. Mittels des Hörers 22, der ein elektromechanischer Schallwandler ist, wird bei Betrieb ein mittels der Signalverarbeitungseinheit 20 bereitgestelltes (elektrisches) Signal in einen Ausgabeschall gewandelt, also in Schallwellen. Diese werden in einen Schallschlauch 24 eingeleitet, dessen eines Ende an dem Gehäuse 14 befestigt ist. Das andere Ende des Schallschlauchs 24 ist mittels eines Doms 26 umschlossen, der im bestimmungsgemäßen Zustand in einem hier nicht näher dargestellten Gehörgang des Trägers 2 des Hörgeräts 4, angeordnet ist.

Innerhalb des Gehäuses 14 ist ferner ein Sensor 28 angeordnet, der ein Beschleunigungssensor ist oder diesen zumindest umfasst. Somit ist es möglich, mittels des Sensors 28 eine Beschleunigung des Gehäuses 14 zu messen und hieraus auch, insbesondere mittels Integration, eine aktuelle Position des Gehäuses 14 zu ermitteln. Auch ist es dabei möglich, eine Neigung des Gehäuses 14 und daher auch eines Kopfes des Trägers 2 zu bestimmen. Innerhalb des Gehäuses 14 ist zudem ein GPS-Sensor 30 angeordnet, anhand dessen eine aktuelle Position des Hörgeräts 4 und somit auch des Trägers 2 bestimmt werden kann. Zudem ist innerhalb des Gehäuses 14 eine Kommunikationsvorrichtung 32 angeordnet, die einem Bluetooth-Standard genügt, und mittels derer die Herstellung der Bluetooth - Verbindung mit dem externen Gerät 8 erfolgt.

Die Bestromung der Signalverarbeitungseinheit 20 erfolgt mittels einer in dem Gehäuse 4 angeordneten Batterie 34. Von der Signalverarbeitungseinheit 20 wird ein Teil der elektrischen Energie zu dem Sensor 28, dem GPS-Sensor 30 sowie der Kommunikationsvorrichtung 32 geleitet. Auch werden mittels dieser elektrischen Energie das Mikrofon 16 sowie der Hörer 22 betrieben.

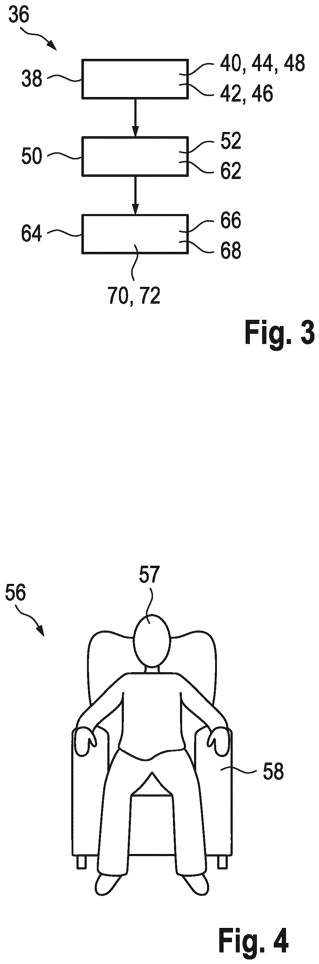

Das Hörgerät 4 ist gemäß einem in Figur 3 dargestellten Verfahren 36 betrieben, wobei das Verfahren 36 zumindest teilweise mittels der Signalverarbeitungseinheit 20 durchgeführt wird, die somit ein Steuergerät oder eine Steuereinheit des Hörgeräts 4 bildet. In einem ersten Arbeitsschritt 38 werden mittels des Sensors 28 Messdaten 40 erstellt. Mit anderen Worten werden eine Beschleunigung und Position des Hörgeräts 4 ermittelt und hierauf auf eine etwaige körperliche Aktivität des Trägers 2 geschlossen. Mit anderen Worten wird überprüft, ob der Träger 2 eine körperliche Aktivität durchführt oder durchgeführt hat, wofür die Messdaten 40 entsprechend realisiert werden. Auch wird anhand der Messdaten 40 eine aktuelle Körperhaltung 42 des Trägerbands 2 ermittelt. Mit anderen Worten wird ermittelt, ob der Träger 2 aktuell steht, sitzt oder läuft, und wie die Haltung des Kopfes des Trägers 2 ist sowie gegebenenfalls auch des Oberkörpers ist.

Ferner werden mittels des Mikrofons 16 weitere Messdaten 44 erstellt. Die weiteren Messdaten 44 korrespondieren hierbei zu dem Schall, der zu den einzelnen Mikrofoneinheiten 18 gelangt und mittels derer registriert wird. Anhand der weiteren Messdaten 44 wird ein die Atmung des Trägers 2 charakterisierende Wert 46 ermittelt. Der charakterisierende Wert 46 umfasst eine Atemfrequenz, des Trägers 2. Hierfür werden die Atemgeräusche des Trägers 2, die in den weiteren Messdaten 44 vorliegen, entsprechend analysiert. Auch umfasst der charakterisierende Wert 46 eine Tiefe dessen Einatmens, sowie die Information, ob dessen Ausatmen durch zusammengepresst Lippen erfolgt. Auch hierauf werden die weiteren Messdaten 44 entsprechend analysiert.

Zudem wird in dem ersten Arbeitsschritt 38 mittels des GPS-Sensors eine aktuelle Position des Hörgeräts 4 und somit auch des Trägers 2 des Hörgeräts 4 ermittelt. Auch werden über die Bluetooth-Verbindung von dem externen Sensor 10 externe Daten 48 empfangen, die mittels des Kommunikationsbausteins des externen Geräts 8 übermittelt werden. Die externen Daten 48 betreffen den körperlichen Zustand des Trägers 2 und entsprechend dessen Pulsfrequenz.

In einem sich anschließenden zweiten Arbeitsschritt 50 wird anhand der Messdaten 40 und der weiteren Messdaten 44 auf eine Atembeschwerde 52 des Trägers 2 geschlossen. Auch wird eine Schwere der etwaigen Atembeschwerde 52 ermittelt. Zum Schließen auf die Atembeschwerde 52 wird ein in die Signalverarbeitungseinheit 20 integriertes neuronales Netz 54 verwendet. So liegt beispielsweise die Atembeschwerde 52 vor, wenn die aktuelle Körperhaltung 42 im Wesentlichen einer bestimmten Körperhaltung 56 entspricht oder zumindest ähnlich zu dieser ist, und die für eine bestimmte Zeitspanne gehalten wird. Bei jeder bestimmten Körperhaltungen 56 wird die Atmung des Trägers 2 unterstützt, indem bestimmte Muskelgruppen aufgrund der Haltung vorgespannt sind. Auch wird teilweise ein Druck auf den Bauchbereich ausgeübt, sodass die hierfür benötigte erforderlichen Muskeln zum Atmen unterstützt sind.

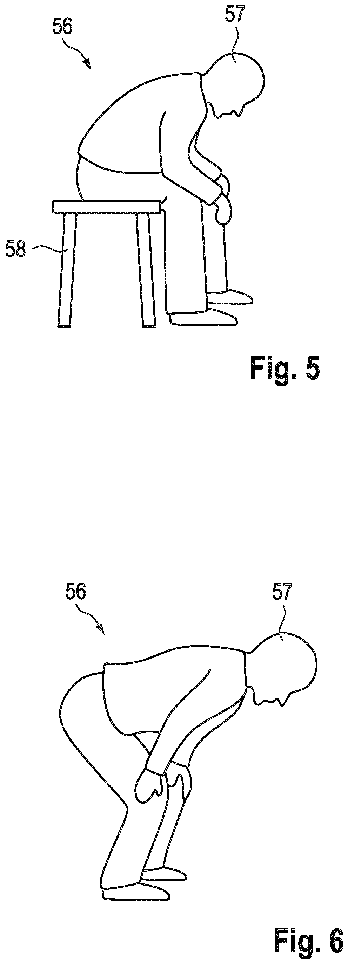

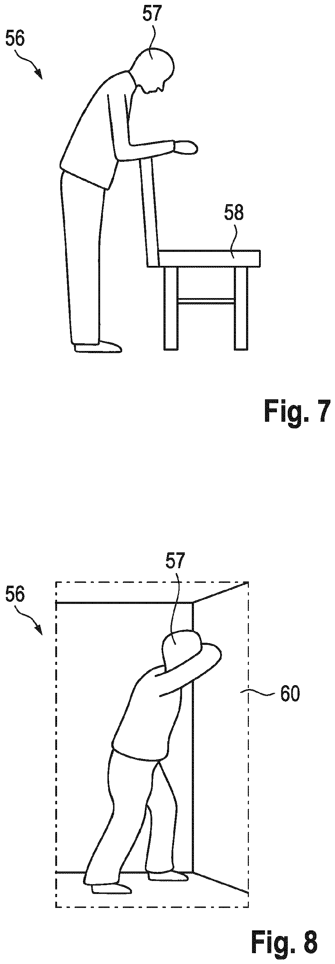

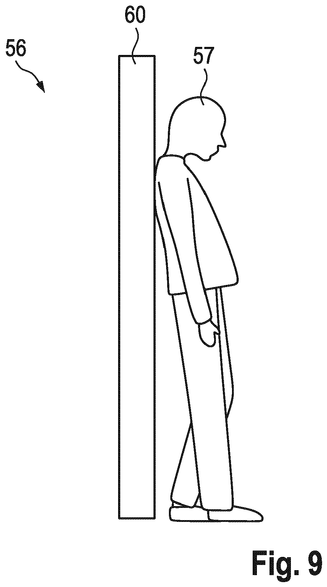

Eine derartige bestimmte Körperhaltung 56 ist der sogenannte Paschasitz, der in Figur 4 dargestellt ist. Hierbei sitzt die jeweilige Person 57 mit durchgedrücktem Rücken auf einem Sitzmöbel 58. Der Kopf der Person 57 ist dabei leicht nach unten geneigt. Eine andere der bestimmten Körperhaltungen 56 ist in Figur 5 schematisch dargestellt und entspricht dem sogenannten Kutschersitz. Hierbei sitzt die Person 57 auf dem Sitzmöbel 58, wobei der Oberkörper nach vorne geneigt und die Ellbogen auf den Oberschenkeln abgestützt sind. Der Kopf der Person 57 ist nach unten gerichtet. Bei einer weiteren derartigen bestimmten Körperhaltung 56, die in Figur 6 dargestellt ist, geht die Person 57 in die Hocke, wobei die Beine gespreizt sind. Die Hände sind auf den Oberschenkeln oder den Knien abgestützt. Der Kopf der Person 57 ist nach unten gerichtet. Bei einer weiteren Variante, die in Figur 7 und Figur 8 dargestellt ist, ist der Oberkörper der Person 57 nach vorne geneigt, wobei die Unterarme entweder auf eine Lehne des Sitzmöbels 58 oder einer Wand 60 abgestützt sind. Der Kopf der Person 57 ist jeweils nach unten geneigt. Bei einer weiteren Variante der bestimmten Körperhaltung 56, die in Figur 9 dargestellt ist, ist der Oberkörper der Person 57 an einer Wand 60 angelehnt, wobei der Kopf nach unten geneigt ist.

Bei Vorliegen der Atembeschwerde 52 wird von dem Träger 2 unbewusst eine der bestimmten Körperhaltungen 56 eingenommen, sodass dessen Atmen unterstützt wird. Mittels Analyse, ob die aktuelle Körperhaltung 42 zu einer der bestimmten Körperhaltung 56 korrespondiert, ist ein Anhaltspunkt auf das Vorliegen der Atembeschwerde 52 gegeben. Zum Überprüfen, ob die aktuelle Körperhaltung 42 einer der bestimmten Körperhaltungen 56 entspricht, wird das neuronale Netz 54 verwendet.

Mittels des neuronalen Netzes 46 wird hierbei die jeweils aktuelle Körperhaltung 42 im Hinblick auf eine der bestimmten Körperhaltungen 56 im Zusammenspiel mit dem charakterisierenden Wert 46 bewertet und daraus abgeleitet, ob die Atembeschwerde 20 vorliegt. So wird beispielsweise bei gleichem charakterisierenden Wert 46 jedoch unterschiedlichen aktuellen Körperhaltungen 42 unterschiedlich auf die Atembeschwerde 52 geschlossen. Auch wird beispielsweise, wenn die aktuelle Körperhaltung 42 einer der bestimmten Körperhaltungen 56 entspricht, sich jedoch die charakterisierenden Werte 46 unterscheiden, unterschiedlich auf die Atembeschwerde 52 geschlossen, also ob diese vorliegt oder nicht. Zusammenfassend wird somit anhand der aktuellen Körperhaltung 42 des Trägers 2 sowie anhand des charakterisierenden Werts 46 auf die Atembeschwerde 52 geschlossen, wofür das neuronale Netz 54 verwendet wird. Zudem wird die Schwere der Atembeschwerde 52 ermittelt. Das neuronale Netz 54 ist bereits herstellerseitig angelernt, wird jedoch während des Tragens an den Träger 2 angepasst, beispielsweise kontinuierlich oder in einer Anlernphase.

Sobald darauf geschlossen wurde, dass die Atembeschwerde 52 vorliegt, also dass der Träger 2 unter der Atembeschwerde 52 leidet, wie aktueller Luftnot, wird ein Maß 62 für ein Risiko ermittelt. Beim Ermitteln des Maßes 62 wird die ermittelte Schwere der Atembeschwerde 52 herangezogen. Auch wird beim Ermitteln des Maßes 62 berücksichtigt, ob die körperliche Aktivität des Trägers 2 vorliegt oder vorlag. Somit ist das Maß 62 reduziert, wenn der Träger 2 vor dem Schließen auf die Atembeschwerde 52 eine schwere körperliche Aktivität durchführte. Auch werden die externen Daten 48, also die Pulsfrequenz beim Ermitteln des Maßes 62 verwendet. So wird bei einem vergleichsweise hohen Puls und vorliegenden Atembeschwerden 52, wobei keine körperliche Aktivität stattfand, ein hohes Maß 62 verwendet. Dahingegen wird bei einem verringerten Puls und/oder wenn eine körperliche Aktivität des Trägers 2 stattfindet oder stattfand ein verringertes Maß 62 verwendet.

In einem sich anschließenden dritten Arbeitsschritt 64 wird eine aktuelle Umgebungssituation 66 ermittelt. Hierfür werden die weiteren Messdaten 44 auf Hintergrundsignale überprüft, wie sich unterhaltende Personen. Auch wird die mittels des GPS-Sensors 30 ermittelte Position mit einem in der Signalverarbeitungseinheit 20 hinterlegten Karte verglichen und folglich der Ort ermittelt, an dem sich der Träger 2 aufhält. So wird beispielsweise überprüft, ob sich der Träger 2 zu Hause, in einem Verkehrsmittel oder beispielsweise auf einer kulturellen Veranstaltung, wie bei einem Opernbesuch aufhält.

In Abhängigkeit der aktuellen Umgebungssituation 66 sowie in Abhängigkeit des Maßes 62 für das Risiko wird eine dem Träger 2 helfende Tätigkeit 68 durchgeführt, die auch lediglich als Tätigkeit bezeichnet wird. Als helfende Tätigkeit 68 wird bei einem hohen Maß 62 das Aussenden einer Notfallmeldung 70 mittels der Kommunikationsvorrichtung 32 herangezogen. Bei einem derart hohen Maß 62 liegt eine lebensbedrohliche Situation für den Träger 2 vor, sodass dieser auf zusätzliche Hilfe angewiesen ist. Die Notfallmeldung 70, in der auch die mittels des GPS-Sensors 30 ermittelte Position hinterlegt ist, wird zu dem externen Gerät 8 gesandt, mittels dessen die Notfallmeldung 70 mittels des Kommunikationsbausteins zu einer Notrufzentrale oder Rettungsstellen weitergeleitet wird, sodass medizinisches Notfallpersonal zu dem Träger 2 gelangen kann. Auch wird beispielsweise das Mikrofon 16 oder der Hörer 22 zum Ausgeben von Schall angesteuert, sodass sich in der Nähe des Trägers 2 befindende Personen auf die Situation des Trägers 2 aufmerksam gemacht werden.

Falls das Maß 62 geringer ist, wird als helfende Tätigkeit 68 eine Ausgabe einer Anweisung 72 herangezogen. Hierfür wird der Hörer 22 entsprechend angesteuert, sodass die Anweisung 72 von dem Träger 2 wahrgenommen werden kann. Die Anweisung 72 betrifft die Einnahme einer die Atmung verstärkt unterstützenden Körperhaltung 56. Sofern der Träger 2 bereits eine der bestimmten Körperhaltungen 56 eingenommen hat, wird zunächst überprüft, welche der bestimmten Körperhaltungen 56 die Atmung in einem höheren Maß unterstützt. Nachfolgend wird diese mittels der Anweisung 72 dem Träger 2 mitgeteilt, wofür beispielsweise entweder der Name der bestimmten Körperhaltungen 56 oder die Anordnung der Extremitäten, wie der Arme, ausgegeben wird.

Die Erfindung ist nicht auf das vorstehend beschriebene Ausführungsbeispiel beschränkt. Vielmehr können auch andere Varianten der Erfindung von dem Fachmann hieraus abgeleitet werden, ohne den Gegenstand der Erfindung zu verlassen. Insbesondere sind ferner alle im Zusammenhang mit dem Ausführungsbeispiel beschriebene Einzelmerkmale auch auf andere Weise miteinander kombinierbar, ohne den Gegenstand der Erfindung zu verlassen.

### Bezugszeichenliste

- 2: Träger
- 4: Hörgerät
- 6: Ohr
- 8: externes Gerät
- 10: externer Sensor
- 12: System
- 14: Gehäuse
- 16: Mikrofon
- 18: Mikrofoneinheit
- 20: Signalverarbeitungseinheit
- 22: Hörer
- 24: Schallschlauch
- 26: Dom
- 28: Sensor
- 30: GPS-Sensor
- 32: Kommunikationsvorrichtung
- 34: Batterie
- 36: Verfahren
- 38: erster Arbeitsschritt
- 40: Messdaten
- 42: Körperhaltung
- 44: weitere Messdaten
- 46: charakterisierender Wert
- 48: externe Daten
- 50: zweiter Arbeitsschritt
- 52: Atembeschwerde
- 54: neuronales Netz
- 56: bestimme Körperhaltung
- 57: Person
- 58: Sitzmöbel
- 60: Wand
- 62: Maß
- 64: dritter Arbeitsschritt
- 66: aktuelle Umgebungssituation
- 68: helfende Tätigkeit
- 70: Notfallmeldung
- 72: Anweisung

## Patentansprüche

1. Verfahren (36) zum Betrieb eines Hörgeräts (4), das einen Sensor (28), ein Mikrofon (16) und einen Hörer (22) aufweist, bei welchem
- anhand von mittels des Sensors (28) erstellten Messdaten (40) auf eine Atembeschwerde (52) eines Trägers (2) geschlossen und basierend hierauf ein Maß (62) für ein Risiko ermittelt wird,
- in Abhängigkeit des Maßes (62) eine dem Träger (2) helfende Tätigkeit (68) durchgeführt wird.

2. Verfahren (36) nach Anspruch 1,
**dadurch gekennzeichnet,**
**dass** anhand der Messdaten (40) eine aktuelle Körperhaltung (42) des Trägers (2) ermittelt und hieraus auf die Atembeschwerde (52) geschlossen wird.

3. Verfahren (36) nach Anspruch 1 und 2,
**dadurch gekennzeichnet,**
**dass** anhand von mittels des Mikrofons (16) erstellten weiteren Messdaten (44) ein die Atmung des Trägers (2) charakterisierender Wert (46) ermittelt und zum Schließen auf die Atembeschwerde (52) verwendet wird.

4. Verfahren (36) nach einem der Ansprüche 1 bis 3,
**dadurch gekennzeichnet,**
**dass** zum Schließen auf die Atembeschwerde (52) ein neuronales Netz (54) verwendet wird.

5. Verfahren (36) nach einem der Ansprüche 1 bis 4,
**dadurch gekennzeichnet,**
**dass** eine ermittelte körperliche Aktivität des Trägers (2) beim Ermitteln des Maßes (62) berücksichtigt wird.

6. Verfahren (36) nach einem der Ansprüche 1 bis 5,
**dadurch gekennzeichnet,**
**dass** von einem externen Sensor (10) externe Daten (48) betreffend den körperlichen Zustand des Trägers (2) empfangen und beim Ermitteln des Maßes (62) verwendet werden.

7. Verfahren (36) nach einem der Ansprüche 1 bis 6,
**dadurch gekennzeichnet,**
**dass** eine aktuelle Umgebungssituation (66) ermittelt und auch in Abhängigkeit hiervon die helfende Tätigkeit (68) durchgeführt wird.

8. Verfahren (36) nach einem der Ansprüche 1 bis 7,
**dadurch gekennzeichnet,**
**dass** die helfende Tätigkeit (68) eine Ausgabe einer Anweisung (72) zur Einnahme einer die Atmung verstärkt unterstützenden Körperhaltung (56) umfasst.

9. Verfahren (36) nach einem der Ansprüche 1 bis 8,
**dadurch gekennzeichnet,**
**dass** die helfende Tätigkeit (68) das Aussenden einer Notfallmeldung (70) mittels einer Kommunikationsvorrichtung (32) umfasst.

10. Hörgerät (4), insbesondere Hörhilfegerät, welches einen Sensor (28), ein Mikrofon (16) und einen Hörer (22) aufweist, und welches gemäß einem Verfahren (36) nach einem der Ansprüche 1 bis 9 betrieben ist.
